(19) Europäisches Patentamt
European Patent Office
Office européen des brevets

(11) Numéro de publication: **0 122 855**
**B1**

(12) # FASCICULE DE BREVET EUROPÉEN

(45) Date de publication du fascicule du brevet: **12.08.87**

(21) Numéro de dépôt: **84400712.0**

(22) Date de dépôt: **11.04.84**

(51) Int. Cl.⁴: **C 07 D 401/04**, C 07 D 405/14, C 07 D 403/04, C 07 D 409/14, A 61 K 31/53

(54) **Piperidines disubstitutées, leurs procédés de préparation et les compositions pharmaceutiques les renfermant.**

(30) Priorité: **12.04.83 FR 8305904**

(43) Date de publication de la demande: **24.10.84 Bulletin 84/43**

(45) Mention de la délivrance du brevet: **12.08.87 Bulletin 87/33**

(84) Etats contractants désignés: **AT BE CH DE FR GB IT LI LU NL SE**

(56) Documents cité:
**EP-A-0 090 733**
**FR-A-2 019 646**

**Le dossier contient des informations techniques présentées postérieurement au dépôt de la demande et ne figurant pas dans le présent fascicule.**

(73) Titulaire: **ADIR, 22, rue Garnier, F-92200 Neuilly- sur-Seine (FR)**

(72) Inventeur: **Régnier, Gilbert, Avenue du Plessis, F-92290 Châtenay- Malabry (FR)**
Inventeur: **Dhainaut, Alain, 7 Rue des Guipières, F-78400 Chatou (FR)**
Inventeur: **Laubie, Michel, 35 Avenue Foch, F-92420 Vaucresson (FR)**
Inventeur: **Duhault, Jacques, 14 Bis rue P. Demange, F-78290 Croissy- sur- Seine (FR)**
Inventeur: **Roman, François, 10 Rue de Dieppe, F-92400 Courbevoie (FR)**

EP 0 122 855 B1

Il est rappelé que: Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

LIBER, STOCKHOLM 1987

**Description**

La présente invention a pour objet des piperidines disubstituées, leurs procédés de préparation et les compositions pharmaceutiques les renfermant.

Elle concerne particulièrement les composé de formule générale (I):

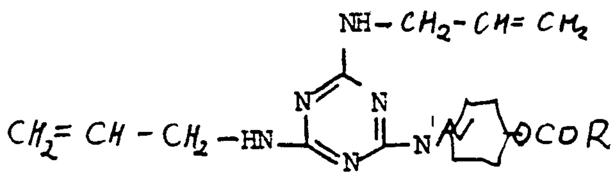

I

dans laquelle:

R représente:

- un radical hydrocarboné ayant de 1 à 6 atome de carbone en chaîne droite ou ramifiée, comportant éventuellement un atome d'oxygène ou de soufre, ou une double liaison et éventuellement substitué par un radical phenyle lui-même éventuellement substitué par un ou plusieurs atomes d'halogène ou radicaux alcoxy ayant de 1 à 5 atomes de carbone ou un radical $-O-(CH_2)_m-O-$ dans lequel m représente 1, 2 ou 3;

- un radical phényle eventuellement substitué par un ou plusieurs atomes d'halogène, radicaux alcoxy ayant de 1 à 5 atomes de carbone ou un radical $-O-(CH_2)_m-O-$ dans lequel m représente 1, 2 ou 3; ou

- un radical furyle, thiényle, benzofuryle, benzothiényle, benzodioxannyle, ou benzodioxinnyle; ou

- un radical de formule

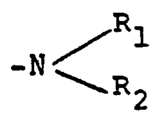

dans laquelle $R_1$ et $R_2$ identiques ou différents représentent chacun un atome d'hydrogène, un radical alcoyle ayant de 1 à 6 atomes de carbone, ou $R_1$ et $R_2$ forment ensemble avec l'atome d'azote auquel ils sont liés un radical hétérocyclique penta ou hexagonal renfermant eventuellement un atome d'oxygène, comme par exemple le radical pyrrolidinyle, pipéridino ou morpholino

L'état antérieur de la technique le plus proche de la présente demande est illustré par FR-A-2.019.646 qui décrit, entre autres, des dérivés de la s.triazine de formule

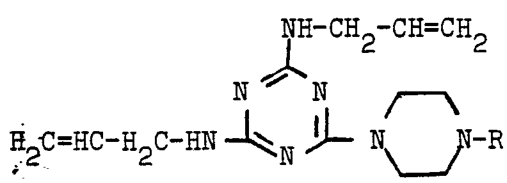

dans laquelle R peut être hydrogène ou un radical hydrocarboné, mais jamais un radical, carbonyloxy-O-COR; lesquels dérivés agissant sur les récepteurs périphériques de la respiration, augmentent la ventilation respiratoire et sont de ce fait utilisables comme analeptique respiratoire.

Le remplacement dans ces dérivés du substituant pipérazinyl 4-substitué

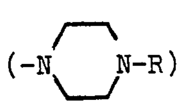

par un radical pipéridinyl 4-substitué

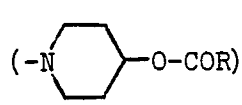

conduit aux dérivés de formule (I) objet de la présente demande qui favorisent la captation d'oxygène et sont de ce fait utilisables dans le traitement de tout type d'hypoxie tissulaire.

La présente invention a également pour objet le procédé de préparation des dérivés de formule générale (I) caractérisé en ce que l'on condense:

une piperdine de formule générale (II):

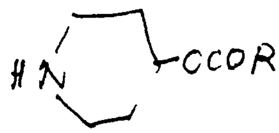

dans laquelle R a la signification énoncée précédemment,

avec un dérivé halogéné de formule générale (III):

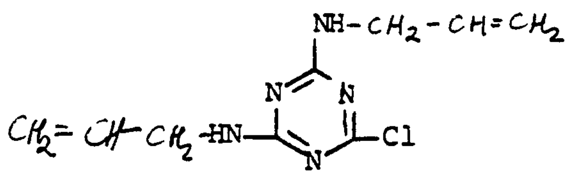

III

La condensation s'effectue de préférence dans un alcool tel que le butanol ou un amide aliphatique comme le diméthylformamide. Il est avantageux d'opérer à une temperature comprise entre 120 et 140° C, en présence d'un accepteur de l'hydracide formé au cours de la réaction. Cet accepteur peut être, par exemple, la triéthylamine ou un excès de la polyméthylène imine II utilisée pour la condensation.

La présente invention a aussi pour objet le procédé de préparation des dérivés de formule générale (I) caractérise en ce que l'on condense:

un composé de formule générale (IV):

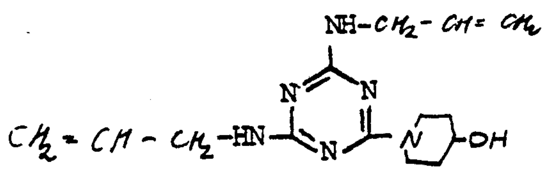

IV

avec un dérivé halogéné de formule générale (V):

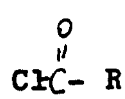

V

dans laquelle R a la signigication enoncée précédemment.

Il est particulièrement avantageux d'effectuer la condensation dans un solvant approprié comme par exemple la pyridine, le tétrahydrofuranne ou le diméthylformamide à une temperature comprise entre 20 et 50° C, en présence d'un accepteur de l'hydracide formé au cours de la réaction. Comme accepteur on peut citer par exemple la triéthylamine ou la pyridine.

Ces nouveaux dérivés ainsi obtenus peuvent être transformés en sels d'addition avec les acides, sels qui font, à ce titre, partie de l'invention. Comme acides utilisables pour la formation de ces sels, on peut citer, par exemple, dans la série minérale les acides chlorhydrique, bromhydrique, sulfurique, phosphorique et dans la série organique, les acides acétiqe, propionique, maléique, fumarique, tartrique, nitrique, oxalique, benzoïque, méthane sulfonique et iséthionique.

Ces nouveaux dérivés peuvent être purifiés par des méthodes physiques telles que cristallisation, chromatographie ou chimiques telles que formation de sels d'addition avec des acides et décomposition de ces sels par des agents alcalins.

Les matières premières utilisées dans les procédes précédemment décrits sont soit des produits connus, soit des produits préparés à partir de substances connues, selon des procédés décrits pour préparer des produits analogues comme indiqué dans les exemples suivants.

Les dérivés de formule générale (I) et leurs sels d'addition physiologiquement tolérables possèdent des propriétés pharmacologiques et thérapeutiques intéressantes. En particulier, ils favorisent la captation

d'oxygène et permettent ainsi leur utilisation comme médicament, notamment dans le traitement de tout type d'hypoxie tissulaire.

Ces dérivés et leurs sels physiologiquement tolérables présentent de plus une très faible toxicité.

L'effet des dérivés de l'invention sur la pression d'oxygène (P $O_2$) a été étuidé chez le chien anesthésié au Nembutal. Des échantillons de sang sont prélevés périodiquement 2, 5, 15, 45 et 75 minutes après l'administration des composés à tester; ils servent à la détermination du pH, de la P $O_2$ et de la P $CO_2$.

La P $O_2$ est mesurée sur un appareil Radiometer $BMS_3$. La lecture de la P $O_2$ se fait sur cet appareil préalablement étalonné avec des valeurs connues, à l'aide d'une électrode de platine, ou électrode de Clark.

Les produits de la présente invention se sont révélés également actifs dans le traitement de l'hypoxie anémique induite par voie chimique par injection souscutanée de $NaNO_2$ selon la méthode de Gibson G.E. Neurobiol aging 2, 165, (1981) et Biochem. Pharmacol, 28, 747, (1979), et dans l'hypoxie hypobare selon la technique de Legeai J.M. et coll. Experientia, 37, 292 (1981).

La présente invention a également pour objet les compositions pharmaceutiques contenant comme principe actif un dérivé de formule générale (I) ou un de ses sels physiologiquement tolérable, mélangé ou associé à un excipient pharmaceutique approprié.

Elle concerne notamment les formes dosées.

Les compositions pharmaceutiques ainsi obtenues sont présentées avantageusement sous des formes dosées diverses telles que par exemple, comprimés, dragées, gélules, suppositoires, solutions injectables ou buvables.

Les exemples suivants illustrent l'invention.

**Exemple 1**:

(bis allylamino-4,6 s.triazinyl-2)-1 acétoxy-4 pipéridine:

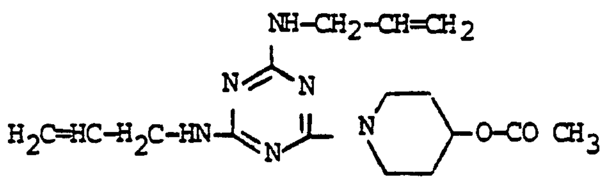

Première méthode:

On chauffe pendant 5 heures à 110°C, une solution de 10 g de bis allylamino-4,6 chloro-2 triazine et de 8 g de chlorhydrate d'acétoxy-4 piperidine, fondant (Kofler) à 160°C, dans 150 ml de n-butanol en présence de 9 g de triéthylamine. Ensuite, on évapore le solvant sous pression réduite et reprend le résidu avec de l'eau. On extrait l'insoluble avec $CHCl_3$. Après évaporation de la solution chloroformique, le résidu est dissout dans un mélange de dichlorométhane-acétate d'éthyle (7/3) puis chromatographié sur silice sous une pression de 101.000 Pa. On recueille 9 g de base pure résineuse que l'on transforme en fumarate au sein de l'éthanol. On isole finalement 10 g de fumarate de bis allylamino-4,6 s.triazinyl-2)-1 acétoxy-4 pipéridine, sous forme de cristaux blancs, fondant (Kofler) à 163°C. Le chlorhydrate d'acétoxy-4 piperidins a été préparé par hydrogénolyse de l'acétoxy-4 benzyl-1 pipéridine Eb/0,15 mmHg 125-130°C, elle même préparée par acétylation pyridinée du benzyl-1 pipéridinol-4.

Deuxième méthode:

On agite à température ambiante pendant 17 heures une solution de 8,7 g de (bis allylamino-4,6 s.triazinyl-2)-1 pipéridinol-4, fondant (Kofler) à 130°C, dans 90 ml de pyridine en présence de 5 g de chlorure d'acétyle. Ensuite, on chasse la pyridine sous pression réduite, reprend le résidu à l'eau et au choroforme, lave abondamment la phase chloroformée à l'eau et l'évapore sous pression réduite. L'huile brune ainsi obtenue (9,7 g) est dissoute dans 100 ml d'éthanol à reflux et on forme le fumarate par addition de 7 g d'acide fumarique. Après cristallisation, on recueille 7,6 g de fumarate de (bis allylamino-4,6 s.triazinyl-2)-1 acétoxy-4 pipéridine, sous forme de cristaux blancs fondant (Kofler) à 163°C. Le (bis allylamino-4,6 s.triazinyl-2)-1 pipéridinol-4 de départ a été préparé par condensation de la bis allylamino-4,6 chloro-2 triazine avec l'hydroxy-4 pipéridine, dans le butanol à 116°C en présence de carbonate de potassium.

**Exemple 2 à 13**:

Les dérivés suivants ont été préparés selon les méthodes décrites dans l'exemple 1:

2. (bis allylamino-4,6 s.triazinyl-2)-1 propionyloxy-4 pipéridine, dont le fumarate fond (capillaire) à 165-167°C

(éthanol anhydre).

3. (bis allylamino-4,6 s.triazinyl-2)-1 benzoyloxy-4 pipéridine, dont le fumarate fond (capillaire) à 135-138° C (éthanol anhydre).

4. (bis allylamino-4,6 s.triazinyl-2)-1 (méthylènedioxy-3,4 benzoyloxy)-4 pipéridine, dont le fumarate fond (capillaire) à 134-137° C (éthanol anhydre).

5. (bis allylamino-4,6 s.triazinyl-2)-1 cinnamoyloxy-4 pipéridine, dont le fumarate fond (capillaire) à 149-153 C (éthanol anydre).

6. (bis allylamino-4,6 s.triazinyl-2)-1 (méthoxy-3 propionyloxy)-4 pipéridine, dont le fumarate fond (capillaire) à 152-153° C (éthanol anhydre).

7. (bis allylamino-4,6 s.triazinyl-2)-1 triméthylacétoxy-4 pipéridine, P.F. (capillaire) du fumarate correspondant: 117-180° C (éthanol anhydre).

8. (bis allylamino-4,6 s.triazinyl-2)-1 (p.chlorophénoxyacétoxy)-4 pipéridine, P.F. (capillaire) du fumarate correspondant: 153-155° C (éthanol anhydre).

9. (bis allylamino-4,6 s.triazinyl-2)-1 (furoyl -2oxy)-4 pipéridine, P.F. (capillaire) du fumarate correspondant: 127-129° C (éthanol anhydre).

10. (bis allylamino-4,6 s.triazinyl-2)-1 (benzothiényl-2 carbonyloxy)-4 pipéridine, P.F. (capillaire) du fumarate correspondant: 172-175° C (éthanol anhydre).

11. (bis allylamino-4,6 s.triazinyl-2)-1 (benzodioxanyl-2 carbonyloxy)-4 pipéridine, P.F. (capillaire) du fumarate correspondant: 168-171° C (éthanol anhydre).

12. (bis allylamino-4,6 s.triazinyl-2)-1 méthoxyacétoxy-4 pipéridine, P.F. (capillaire) du fumarate correspondant: 160-162° C (éthanol anhydre).

13. (bis allylamino-4,6 s.triazinyl-2)-1 (benzodioxinyl-6 carbonyloxy)-4 pipéridine, P.F. (capillaire) du fumarate correspondant: 167-170° C (éthanol anhydre).

## Revendications

pour les états contractants: BE, CH, DE, FR, GB, IT, LI, LU, NL, SE.

1. Les piperidines disubstituées de formule générale (I):

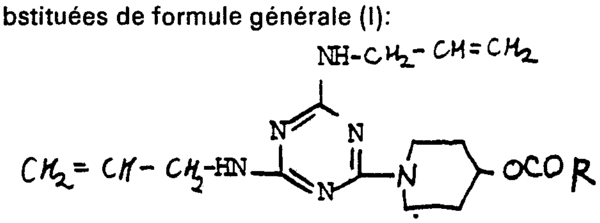

I

dans laquelle R représente:

- un radical hydrocarboné ayant de 1 à 6 atomes de carbone, en chaîne droite ou ramifiée, comportant éventuellement un atome d'oxygène ou de soufre, ou une double laison, et éventuellement substitué par un radical phényle lui-même éventuellement substitué par un ou plusieurs atomes d'halogène ou radicaux alcoxy ayant de 1 à 5 atomes de carbone ou un radical $-O-(CH_2)m-O-$ dans lequel m représente 1, 2 ou 3;
- un radical phényle éventuellement substitué par un ou plusieurs atomes d'halogène, radicaux alcoxy ayant de 1 à 5 atomes de carbone ou un radical $-O-(CH_2)m-O-$ dans lequel m représente 1, 2 ou 3;
- un radical furyle, thiényle, benzofuryle, benzothiényle, benzodioxannyle, ou benzodioxinnyle ou
- un radical de formule

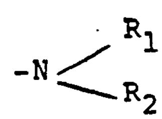

dans laquelle $R_1$ et $R_2$ identiques ou différents représentent chacun un atome d'hydrogène ou un radical alcoyle ayant de 1 à 6 atomes de carbone, ou

$R_1$ et $R_2$ forment ensemble avec l'atome d'azote auquel ils sont liés un radical hétérocyclique penta ou hexagonal renfermant éventuellement un atome d'oxygène; ou

2. Les sels des composés de la revendication 1 avec des acides appropriés.

3. Les sels selon la revendication 2 qui sont physiologiquement tolérables.

4. La (bis allylamino-4,6 s.triazinyl-2)-1 acétoxy-4 pipéridine, et son fumarate.

5. Un procédé de préparation des composés de la revendication 1, caractérisé en ce que l'on condense: une piperidine de formule générale (II):

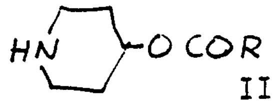

II

dans laquelle R a la signification définie dans la revendication 1, avec un dérivé halogéné de formule générale (III):

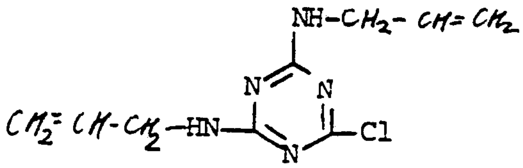

III

6. Un procédé de préparation des composés de la revendication 1, caractérisé en ce que l'on condense: un composé de formule générale (IV):

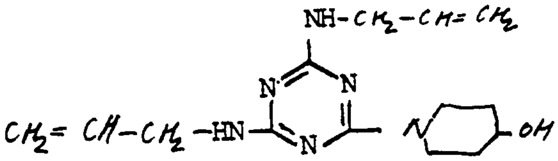

IV

avec un dérivé halogéné de formule générale (V):

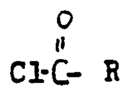

V

dans laquelle R a la signification énoncée dans la revendication 1.

7. Les compositions pharmaceutiques contenant comme principe actif un composé selon les revendications 1, 3 ou 4, avec les excipients pharmaceutiques appropriés.

8. Les compositions pharmaceutiques selon la revendication 7 présentée sous une forme convenant notamment pour le traitement de tout type d'hypoxie tissulaire.

**Revendication**

pour l'état contractant: AT.

Procedé de préparation des piperdines disubstituées de formule générale (I):

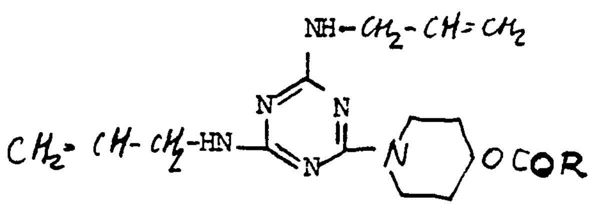

I

dans laquelle R représentant:

- un radical hydrocarboné ayant de 1 à 6 atomes de carbone, en chaîne droite ou ramifiée, comportant éventuellement un atome d'oxygène ou de soufre, ou une double laison, et éventuellement substitué par un radical phényle lui-même éventuellement substitué par un ou plusieurs atomes d'halogène ou radicaux alcoxy ayant de 1 à 5 atomes de carbone ou un radical -O-$(CM_2)$m-O- dans lequel m représente 1, 2 ou 3;
- un radical phényle éventuellement substitué par un ou plusieurs atomes d'halogène, radicaux alcoxy ayant de 1 à 5 atomes de carbone ou un radical -O-$(CH_2)_m$-O- dans lequel m represente 1, 2 ou 3;
- un radical furyle, thiényle, benzofuryle, benzothiényle, benzodioxannyle, ou benzodioxinnyle ou
- un radical de formule

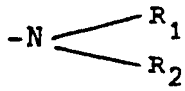

dans laquelle $R_1$ et $R_2$ identiques ou différents représentent chacun un atome d'hydrogène ou un radical alcoyle ayant de 1 à 6 atomes de carbone, ou

$R_1$ et $R_2$ forment ensemble avec l'atome d'azote auquel ils sont liés un radical hétérocyclique penta ou hexagonal renfermant éventuellement un atome d'oxygène

et de leurs sels d'addition avec dss acides appropriés;

caractérisé en ce que l'on condense:

- soit une piperidine de formule générale (II):

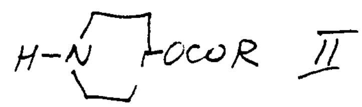

dans laquelle R a la significations précédemment définie avec un dérivé halogéné de formule générale (III):

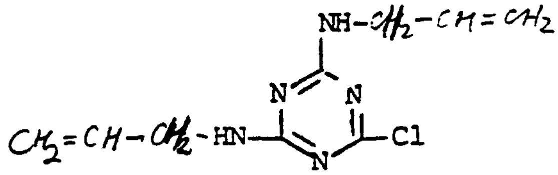

III

- soit une polyméthylène imine de formule générale (IV)

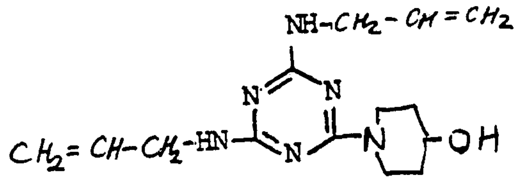

IV

avec un dérivé halogéné de formule générale (V):

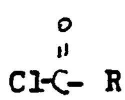

V

dans laquelle R a la signification précédemment définie;

- et si on le désire, on traite les dérivés (I) ainsi obtenus avec les acides appropriés pour donner les sels d'addition correspondants.

**Patentansprüche**

für die Vertragsstaaten: BE, CH, DE, FR, GB, IT, LI, LU, NL, SE.

1. Disubstituierte Piperidine der allgemeinen Formel (I)

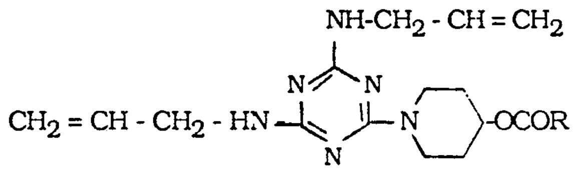

I

in der R

- eine geradkettige oder verzweitgte Kohlenwasserstoffgruppe mit 1 bis 6 Kohlenstoffatomen, die gegebenenfalls ein Sauerstoffatom oder ein Schwefelatom oder eine Doppelbindung aufweist und gegebenenfalls durch eine Phenylgruppe substituiertert ist, die ihrerseits gegebenenfalls durch eines oder mehrere Halogenatome oder Alkoxygruppen mit 1 bis 5 Kohlenstoffatomen oder eine Gruppe der Formel $-O-(CH_2)_m-O-$, in der m den Wert 1, 2 oder 3 besitzt, substituiert ist;

- eine Phenylgruppe, die gegebenenfalls durch eines oder mehrere Halogenatome, Alkoxygruppen mit 1 bis 5 Kohlenstoffatomen oder eine Gruppe der Formel $-O-(CH_2)_m-O-$, in der m den Wert 1, 2 oder 3 besitzt, substituiert ist:

- eine Furylgruppe, eine Thienylgruppe, eine Benzofurylgruppe, eine Benzothienylgruppe, eine Benzodioxanylgruppe oder eine Benzodioxinylgruppe oder

- eine Gruppe der Formel

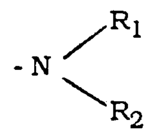

in der $R_1$ und $R_2$, die gleichartig oder verschieden sein können, jeweils Wasserostoffatome oder Alkylgruppen mit 1 bis 6 Kohlenstoffatomen darstellen oder

- $R_1$ und $R_2$ gemeinsam mit dem Stickstoffatom, an das sie gebunden sind, eine fünfgliedrige oder sechsgliedrige heterocyclische Gruppe, die gegebenenfalls ein Sauerstoffatom enthalten kann, bilden: bedeutet.

2. Die Salze der Verbindung nach Anspruch 1 mit geeigneten Säuren.

3. Die Salze nach Anspruch 2, die physiologisch verträglich sind.

4. 1-(4.6-Bis-allylamino-s-triazin-2-yl)-4-acetoxy-piperidin und sein Fumarat.

5. Verfahren zur Herstellung der Verbindungen nach Anspruch 1, dadurch gekennzeichnet, daß man ein Piperidin der allgemeinen Formel (II)

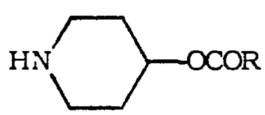

II

in der R die in Anspruch 1 angegebenen Bedeutungen besitzt, mit einem Halogenderivat der allgemeinen Formel (III)

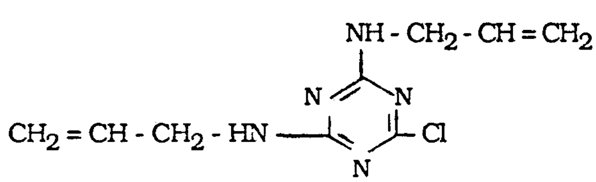

III

kondensiert.

6. Verfahren zur Herstellung der Verbindungen nach Anspruch 1, dadurch gekennzeichnet, daß man eine Verbindung der allgemeinen Formel (IV):

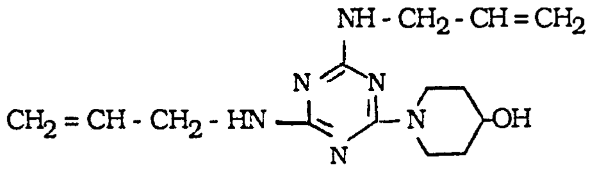

IV

mit einem Halogenderivat der allgemeinen Formel (V):

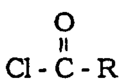

V

in der R die in Anspruch 1 angegebenen Bedeutungen besitzt, kondensiert.

7. Pharmazeutische Zubereitungen enthaltend als Wirkstoff eine Verbindung nnach einem der Ansprüche 1, 3 oder 4 und geeignete pharmazeutische Hilsstoffe.

8. Pharmazeutische Zubereitungen nach Anspruch 7 in einer insbesondere für die Behandlung sämtlicher Arten von Gewebehypoxie geeigneten Form.

**Patentanspruch**

für den Vertragsstaat: AT.

Verfahren zur Herstellung von disubstituierten Piperidinen der allgemeinen Formel (I)

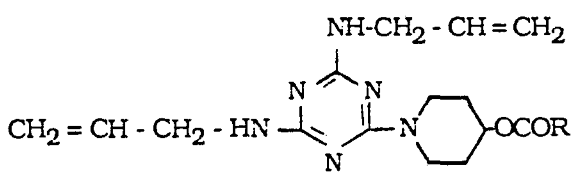

I

in der R
- eine geradkettige oder verzweigte Kohlenwasserstoffgruppe mit 1 bis 6 Kohlenstoffatomen, die gegebenenfalls ein Sauerstoffatom oder ein Schwefelatom oder eine Doppelbindung aufweist und gegebenenfalls durch eine Phenylgruppe substituiert ist, die ihrerseits gegebenefalls durch eines oder mehrere Halogenatome oder Alkoxygruppen mit 1 bis 5 Kohlenstoffatomen oder eine Gruppe der Formel $-O-(CH_2)_m-O-$, in der m den Wert 1, 2 oder 3 besitzt, substituiert ist;
- eine Phenylgruppe, die gegebenenfalls durch eines oder mehrere Halogenatome, Alkoxygruppen mit 1 bis 5 Kohlenstoffatomen oder eine Gruppe der Formel $-O-(CH_2)_m-O-$, in der m den Wert 1, 2 oder 3 besitzt, substituiert ist:
- eine Furylgruppe, eine Thienylgruppe, eine Benzofurylgruppe, eine Benzothienylgruppe, eine Benzodioxanylgruppe oder eine Benzodioxinylgruppe oder
- eine Gruppe der Formel

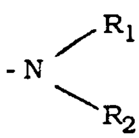

in der $R_1$ und $R_2$, die gleichartig oder verschieden sein können, jeweils Wasserostoffatome oder Alkylgruppen mit 1 bis 6 Kohlenstoffatomen darstellen oder
- $R_1$ und $R_2$ gemeinsam mit dem Stickstoffatom, an das sie gebunden sind, eine fünfgliedrige oder sechsgliedrige heterocyclische Gruppe, die gegebenenfalls ein Sauerstoffatom enthalten kann, bilden:

und ihrer Säureadditionssalze mit geeigneten Säuren, dadurch gekennzeichnet, daß man
- entweder ein Piperidin der allgemeinen Formel (II)

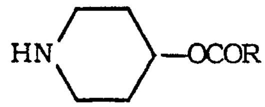

II

in der R die oben angegebenen Bedeutungen besitzt, mit einem Halogenderivat der allgemeinen Formel (III)

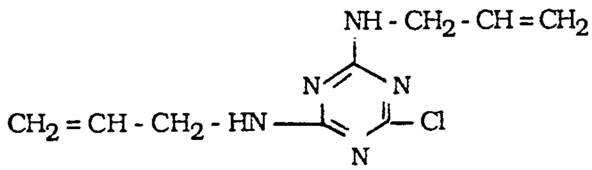

III

kondenstert:
- oder ein Piperidin der allgemeinen Formel (IV)

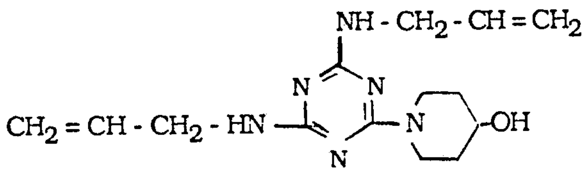

IV

mit einem Halogenderivat der allgemeinen Formel (V):

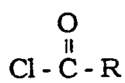

V

in der R die oben angegebenen Bedeutungen besitzt, kondensiert: und
- gewünschtenfalls die auf diese Weise erhaltenen Derivate (I) mit geeigneten Säuren zur Bildung der entsprechenden Additionssalze behandelt.

**Claims**

for the Contracting States BE, CH, DE, FR, GB, IT, LI, LU, NL, SE.

1. Disubstituted piperidines of the general formula (I):

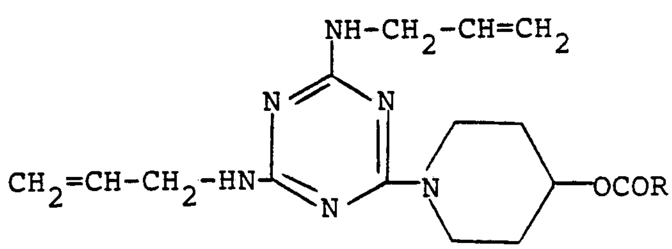

I

in which R represents:
- a straight-chained or branched hydrocarbon radical having from 1 to 6 carbon atoms, optionally having an oxygen or sulphur atom or a double bond and optionally substituted by a phenyl radical that is itself optionally substituted by one or more halogen atoms or one or more alkoxy radicals having from 1 to 5 carbon atoms or by a $-O-(CH_2)_m-O-$ radical in which m represents 1, 2 or 3;
- a phenyl radical optionally substituted by one or more halogen atoms, one or more alkoxy radicals having from 1 to 5 carbon atoms, or by a $-O-(CH_2)_m-O-$radical in which m represents 1, 2 or 3;
- a furyl, thienyl, benzofuryl, benzothienyl, benzodioxanyl or benzodioxinyl radical or
- a radical of the formula

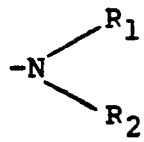

in which $R_1$ and $R_2$ are the same or different and each represents a hydrogen atom or an alkyl radical having from 1 to 6 carbon atoms, or $R_1$ and $R_2$ together form, with the nitrogen atom to which they are bonded, a pentagonal or hexagonal heterocyclic radical optionally containing an oxygen atom.

2. The salts of the compounds of claim 1 with appropriate acids.

3. The salts according to claim 2 that are physiologically tolerable.

4. 1-(4,6-bis-allylamino-2-s.-triazinyl)-4-acetoxy-piperidine and the fumarate thereof.

5. A process for preparing the compounds of claim 1, characterised in that:

a piperidine of the general formula (II):

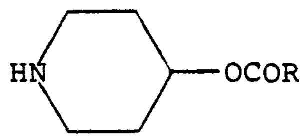

II

in which R has the meaning defined in claim 1, is condensed with a halogenated derivative of the general formula (III):

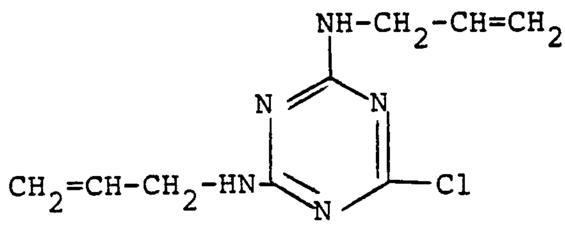

III.

6. A process for preparing the compounds of claim 1, characterised in that:

a compound of the general formula (IV):

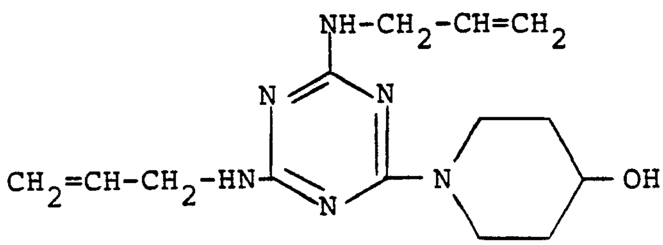

IV,

is condensed with a halogenated derivative of the general formula (V):

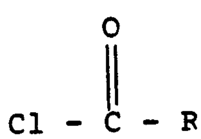

V

in which R has the meaning given in claim 1.

7. Pharmaceutical compositions containing as active ingredient a compound according to claim 1, 3 or 4 with the appropriate pharmaceutical excipients.

8. Pharmaceutical compositions according to claim 7 presented in a form suitable in particular for the treatment of any type of tissue hypoxia.

**Claim**

for the Contracting State AT

Process for the preparation of disubstituted piperidines of the general formula (I):

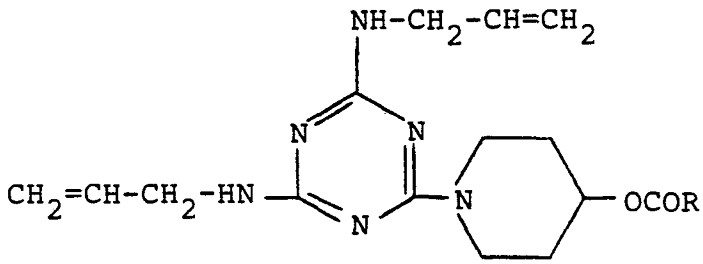

I

in which R represents:

- a straight-chained or branched hydrocarbon radical having from 1 to 6 carbon atoms, optionally having an oxygen or sulphur atom or a double bond and optionally substituted by a phenyl radical that is itself optionally substituted by one or more halogen atoms or one or more alkoxy radicals having from 1 to 5 carbon atoms or by a $-O-(CH_2)_m-O-$ radical in which m represents 1, 2 or 3;
- a phenyl radical optionally substituted by one or more halogen atoms, one or more alkoxy radicals having from 1 to 5 carbon atoms, or by a $-O-(CH_2)_m-O-$radical in which m represents 1, 2 or 3;
- a furyl, thienyl, benzofuryl, benzothienyl, benzodioxanyl or benzodioxinyl radical or
- a radical of the formula

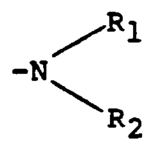

in which $R_1$ and $R_2$ are the same or different and each represents a hydrogen atom or an alkyl radical having from 1 to 6 carbon atoms, or

$R_1$ and $R_2$ together form, with the nitrogen atom to which they are bonded, a pentagonal or hexagonal heterocyclic radical optionally containing an oxygen atom,

and their addition salts with appropriate acids;

characterised in that:

- either a piperidine of the general formula (II):

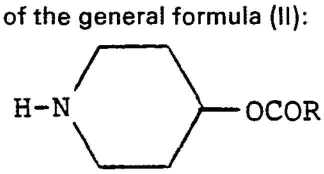

II

in which R has the meaning defined above, is condensed with a halogenated derivative of the formula (III):

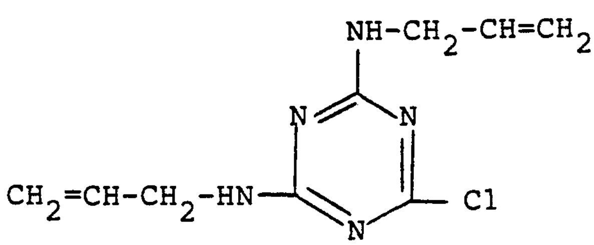

III

- or a piperidine of the formula (IV):

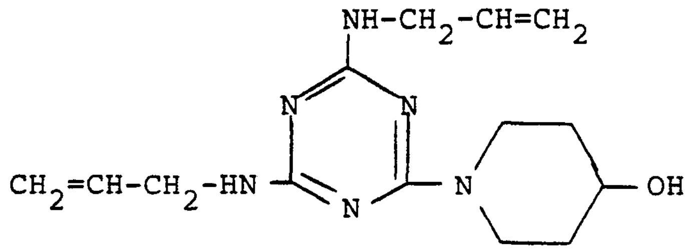

IV,

is condensed with a halogenated derivative of the general formula (V):

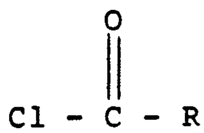

V

in which R has the meaning defined above; and, if desired, the derivatives (I) so obtained are treated with appropriate acids to yield the corresponding addition salts.